# EUROPEAN PATENT APPLICATION

(11) **EP 2 606 879 A1**
(43) Date of publication of application: **26.06.2013**
(21) Application number: 11194984.8
(22) Date of filing: 21.12.2011
(51) Int. Cl.: A61K 9/50, A61K 31/137, A61P 25/04

(54) **Multiple unit pellet tablet formulation comprising an opioid**

(71) Applicant: Hexal AG, 83607 Holzkirchen (DE)
(72) Inventor: Rother, Patrick, 83607 Holzkirchen (DE); Krebeler, Andreas, 83607 Holzkirchen (DE); Täubrich, Theresa, 83607 Holzkirchen (DE); Wawra, Christian, 83607 Holzkirchen (DE); Mohry, Ingeborg, 83607 Holzkirchen (DE)
(74) Representative: Kluschanzoff, Harald

(57) **Abstract**

The present invention relates to a sustained-release pharmaceutical formulation, in particular a multiple unit pellet tablet (MUT) formulation for oral administration comprising a plurality of sustained-release pellets. The invention further relates to sustained-release pellets wherein the active agent is an opioid. The sustained-release pellets are suitable for the preparation of a multiple unit pellet tablet formulation.

## Description

The present invention relates to a sustained-release pharmaceutical formulation, in particular a multiple unit pellet tablet (MUT) formulation for oral administration comprising a plurality of sustained-release pellets. The invention further relates to sustained-release pellets wherein the active agent is an opioid. The sustained-release pellets are suitable for the preparation of a multiple unit pellet tablet formulation.

Sustained-release formulations are desirable in the treatment of a number of diseases because they provide a substantially constant rate of release over an extended period of time thus avoiding plasma peak levels and reducing undesirable side effects. This is particularly important in the treatment of pain in mammals, particularly in humans which can be adequately managed only with opioid analgesics. Opioid analgesics still play a major role in the treatment of acute and chronic pain, particularly pain associated with terminal diseases such as cancer, and degenerative conditions such as rheumatoid arthritis.

An advantage of sustained-release formulations is that they have to be administered less frequently than immediate-release formulations of the same drug, thus enhancing patient compliance. In order to achieve constant analgesia, such formulations can be taken by the patient independently of the presence of a physician and not more frequently than once or twice a day.

In order to achieve a successful and satisfactory treatment of pain for the patients the dose and dosing interval have to be specifically optimized for each patient such that the opioid doses are administered before the pain returns and according to a strictly regular regimen. An individual dosage regimen for each individual patient has to be established according to the patient's physical parameters such as sex, weight, age, physical condition as well as according to the type, duration and severity of the pain condition. It is evident that these parameters result in a complex situation which differs from one individual to another. Thus, in order to establish an individual dosage regimen a certain unit dose determined for an average patient may not be suitable in any case. Instead, a certain proportion of a unit dose may be suitable for a particular patient.

Further, even if a dosage regimen has been established for an individual patient, it may still be required to administer a certain additional dose in between two administrations of the dosage regimen in order to achieve constant analgesia. In such a case it is often not desirable for the patient to take an entire unit dose. Instead it may be suitable to administer only a certain proportion of a unit dose, e.g. one half of a unit dose or one third of a unit dose. Similarly, if the patient recognizes that only a certain proportion of a unit dose may be sufficient, it may be suitable to administer only a certain proportion of a unit dose, e.g. one half of a unit dose or one third of a unit dose.

At the same time, it is crucial to maintain the sustained-release characteristics of the formulation to avoid dose-dumping and to ensure a substantially constant rate of release over an extended period of time. Therefore, there is a need for a dosage form which can be split, preferably into specific predetermined portions, while retaining its sustained-release characteristics.

Several types of sustained-release formulations are known in the art. Frequently, sustained-release formulations are in the form of sustained-release tablets or capsules.

A very simple type of sustained-release formulations are film-coated tablets which, after being pressed, have been coated with a coating chosen to control the rate of dissolution of the drug in the gastrointestinal tract. However, such sustained-release tablets should not be broken before use, as this will expose the tablet core to the digestive juices, thereby circumventing the intended delayed-release effect. This is a major drawback since it is sometimes necessary to split tablets, e.g. into halves or quarters, to achieve individual dosing or dose adjustment.

In another type of sustained-release formulations the active ingredient is embedded in a matrix of one or more insoluble substances such that the dissolving active ingredient is released through the holes in the matrix. For example, EP1439829 discloses a delayed-release pharmaceutical formulation containing the opioid tapentadol or a salt thereof in a matrix containing one or more hydrophilic or hydrophobic polymers. Splitting of such matrix formulations may lead to an alteration of the release kinetics and to a loss of the sustained-release effect. However, some matrix formulations may be cut in half without an influence on the release kinetics.

In an alternative sustained-release formulation the active ingredient is enclosed in polymer-based tablets with a laser-drilled hole on one side and a porous membrane on the other side. Stomach acids enter through the porous membrane, thereby pushing the drug out through the laser-drilled hole. Over the time, the entire drug dose is released into the system while the polymer container remains intact, to be later excreted through normal digestion. A review article by Malaterre et al. (Eur. J. Pharm. Biopharm. 73 (2009) 311-323) provides an overview about oral osmotically driven systems. Also in the case of such osmotic release oral systems (OROS) it is not possible to split the formulation without losing the intended sustained-release effect.

Further, micro-encapsulation can be used to obtain sustained-release formulations. Therefore, an active pharmaceutical ingredient is coated around an inert core and layered with substances providing for sustained-release to form a microsphere or pellet.

EP0377518 discloses a sustained-release pharmaceutical pellet composition for the treatment of pain-associated conditions in patients. Said composition comprises a core element including an active ingredient of high solubility, e.g. a morphine compound, and a core coating for the core element which is partially soluble at a highly acidic pH to provide a slow rate of release of active ingredient such that blood levels of active ingredient are maintained within the therapeutic range over an extended period of time.

WO2010/121600 discloses a pharmaceutical composition comprising first particles and second particles wherein the first particles comprise an opioid and the second particles comprise an opioid antagonist. The particles are each formulated as delayed-release pellets and therefore provide for particular release characteristics. These delayed-release pellets are provided in a sachet or enclosed in a capsule, such as a gelatin capsule.

Active ingredient-containing capsules are known in the art as multiple unit pellet systems (MUPS). Such capsules can be opened and a certain proportion of the enclosed sustained-release pellets can be administered without loss of the intended delayed-release effect. The same is true for sustained-release pellets provided in a sachet. However, it is elaborate and inaccurate to divide the pellets into two or more equal portions in order to achieve individual dosing or dose adjustment.

As can be seen from the prior art, it is usually not possible to split conventional sustained-release formulations into predetermined, defined portions while maintaining their sustained-release characteristics.

In addition to sustained-release pellets provided in a sachet or enclosed in a capsule also MUPS tablets have been described.

EP2057984 discloses a tablet having a tablet core comprising a plurality of hydromorphone-containing pellets and one or more pharmaceutically tolerated excipients and at least one coat applied to the tablet core. The hydromorphone-containing pellets have the following structure: an inert core, an active substance layer applied to the inert core, a layer which is applied to the active substance layer and delays the release of the active substance and a further active substance layer applied on the layer delaying the release of the active substance. This further active substance layer not only provides for a fast release of active substance but also avoids damaging of the release-delaying layer during preparation of the MUPS tablets, which otherwise may result in uncontrolled alterations of the release profile of the pellets.

Several documents known in the art report that direct compression of pellets, comprising a sustained-release layer on top of an active agent layer, optionally in combination with pharmaceutically acceptable excipients in order to obtain tableted pellets results in problems such as damaging of the functional layer, e.g. the sustained-release layer, which may lead to uncontrolled and unreproducible alteration of the release profile, thereby causing considerable risks for the patient.

Prior art pharmaceutical preparations may also suffer from other disadvantages. For example, pharmaceutical sustained-release formulations known in the art often exhibit dose dumping. Dose dumping is a phenomenon characterized by the rapid and unintended release of the entire dose from a sustained release carrier which may occur if the carrier breaks due to chemical stress or dissolves due to chemical reactions. For example, dose dumping may take place if at the same time of taking their medication patients ingest other substances like fatty meals or alcohol that increase drug delivery. Such substances may influence the release rate of the active agent from the sustained-release formulation, or they may stimulate the body's absorptive surfaces to increase the rate of drug uptake. Dose dumping can greatly increase the concentration of an active agent in the patient's body and thereby produce adverse effects or even drug-induced toxicity. Particularly in the case of opioids dose dumping is a severe problem since an opioid overdose may cause severe side effects such as cyanosis, respiratory depression, drop in blood pressure, shock and cramps with often fatal complications.

Another disadvantage frequently observed with prior art pharmaceutical preparations is that bioavailability of prior art preparations may be compromised by food. This is important since complex dosage regimens may lead to non-compliance. A number of sustained-release formulations known in the art exhibit a pronounced food effect.

It was an object of the present invention to overcome at least some of the disadvantages of the sustained-release formulations known in the art. In particular, it was an object of the present invention to provide a sustained-release pharmaceutical formulation which can be divided without loss of the sustained-release characteristics.

The present invention relates to a sustained-release pharmaceutical formulation, in particular a multiple unit pellet tablet (MUT) formulation for oral administration comprising a plurality of sustained-release pellets and one or more pharmaceutically acceptable excipients, wherein the sustained-release pellets comprise as an active agent at least one opioid or a salt thereof.

It was surprisingly found that the multiple unit pellet tablet formulation according to the present invention can be split while retaining its sustained-release characteristics. Further, the MUT formulation of the invention exhibits substantially no food effect or dose dumping and is characterized by an improved reproducibility in vivo.

Sustained-release pharmaceutical formulations have to be administered less frequently than immediate-release formulations of the same drug, e.g. only once or twice a day. This allows achieving constant analgesia while at the same time enhancing patient compliance.

The multiple unit pellet tablet formulation according to the present invention provides a substantially constant rate of release over an extended period of time. According to the invention, the release period of the opioid is at least 6 h, preferably at least 8 h, more preferably at least 12 h, e.g. 12-24 h. Therefore, the multiple unit pellet tablet formulation according to the present invention may be administered at a dosing interval of at least 6 h, preferably at least 8 h, more preferably at least 12 h. Thus, such a formulation is administered only once a day with a dosing interval of approximately 24 h or only twice a day with a dosing interval of approximately 12 h. The multiple unit pellet tablet formulation according to the present invention is preferably administered twice a day with a dosing interval of approximately 12 h.

It is sometimes necessary to split tablets into halves or quarters, e.g. to achieve individual dosing or dose adjustment. Tablets are easier to break accurately if scored, however, devices called pill-splitters can be used to cut unscored and scored tablets.

In the multiple unit pellet tablet formulation of the present invention the active agent is distributed homogenously. Therefore, it is possible to split the dosage unit into two or more equally effective portions.

Importantly, the multiple unit pellet tablets according to the present invention can be split without loss of their sustained-release characteristics. In particular, the multiple unit pellet tablets according to the present invention can be split into portions without loss of their sustained-release characteristics, e.g. into predetermined, defined portions. This can be easily achieved if the multiple unit pellet tablets are scored. Alternatively, this can be achieved using a pill-splitter. Non-limiting examples of predetermined, defined portions of a dosage unit are one half, one third or one quarter.

It is particularly surprising that the MUT formulation of the present invention can be split while retaining its sustained-release characteristics as the sustained-release pellets are not further coated. It has previously been thought that direct compression of sustained-release pellets and one or more pharmaceutically acceptable excipients results, at least partially, in the destruction of the sustained-release layer and, thus, in loss of the sustained-release characteristics of the formulation. Therefore, multiple unit pellet tablets known in the art comprise an additional layer on top of the sustained-release layer in order to protect the sustained-release layer from being damaged during compression. This additional layer is sometimes an additional active agent layer.

According to the present invention, it is not required to apply an additional layer on the sustained-release layer. Therefore, the MUT formulation of the present invention is easier to prepare than MUT formulations known from the prior art.

Monolithic sustained-release dosage forms such as compressed tablets which are provided with a sustained-release coating frequently exhibit dose dumping when split. This represents a severe problem in the case of opioid-containing formulations. Advantageously, the multiple unit pellet tablet formulation according to the present invention shows no evidence of dose dumping.

Similarly, sustained-release formulations known in the art frequently exhibit a pronounced food effect. However, the relative bioavailability of the active agent from the multiple unit pellet tablet formulation according to the present invention is not compromised by food. In particular, the multiple unit pellet tablet formulation exhibits improved reproducibility in vivo. Thus, the blood level curves within a specific patient are highly similar if the same medication is taken on different days regardless of the meals. As a consequence, the inventive MUT may be taken without regard to meals, which further improves compliance. Consequently, concomitant administration of the inventive MUT with food will not significantly decrease the extent of absorption of the active agent or alter the rate of the absorption of the active agent when compared with administration in the fasted state.

Whenever opioid-containing pharmaceutical formulations are concerned there is a high risk of abuse. However, the multiple unit pellet tablet of the present invention significantly reduces the risk of abuse since quantitative extraction of the opioid from the sustained-release pellets is hardly technically feasible.

The sustained-release pellets used in the MUT formulation of the present invention are characterized by the following structure:
a) a neutral core,
b) an active agent layer applied to the neutral core comprising as an active agent at least one opioid or a salt thereof and
c) a sustained-release layer which is applied to the active agent layer.

In particular, no further active agent layer is applied to the sustained-release layer.

The invention further relates to sustained-release pellets wherein the active agent is an opioid. The sustained-release pellets are suitable for the preparation of a multiple unit pellet tablet formulation.

The expressions "multiple unit pellet tablet formulation" or "multiple unit pellet tablet" according to the present invention relate to a pharmaceutical formulation in the form of a compressed tablet, wherein pellets such as the sustained-release pellets according to the invention are compressed together with one or more pharmaceutically acceptable excipients, e.g. by direct compression. Methods for the preparation of multiple unit pellet tablets are known in the art.

The sustained-release pellets of the present invention are prepared by applying an active agent layer to a neutral core, e.g. by means of a fluidized bed pellet-coater. The resulting active agent-containing pellets are further coated with a sustained-release layer, e.g. using a fluidized bed pellet-coater, in order to obtain sustained-release pellets. Said sustained-release pellets are combined with one or more pharmaceutically acceptable excipients and subsequently compressed into tablets, also referred to as tablet cores. These tablet cores may optionally be coated with an additional layer.

The terms "neutral core" or "starter pellets" relate to an active agent-free carrier, onto which the active agent is applied, e.g. by spraying. Examples of a suitable neutral core are pellets such as sugar pellets (nonpareils), cellulosic pellets and the like. According to the invention, said starter pellets are present in the multiple unit pellet tablet formulation in an amount of 0.1-30% by weight based on the total weight of the formulation (w/w), preferably 0.3-17% w/w.

As used herein, the term "active agent layer" refers to a coating comprising the active agent. According to the present invention, the active agent is an opioid agonist, an opioid antagonist or a mixture thereof. Said active agent is applied to the neutral core as the active agent layer in order to obtain active agent-containing pellets.

The active agent-containing pellets are present in the multiple unit pellet tablet formulation in an amount of 15-50% by weight based on the total weight of the formulation (w/w), preferably 30-35% w/w.

As used herein, a "sustained-release layer" is a layer which is applied to the active agent layer and which delays the release of the active agent.

"Sustained-release pellets" according to the present invention are pellets characterized by the following structure: a neutral core, an active agent layer applied to the neutral core and a sustained-release layer which is applied to the active agent layer. According to the present invention the sustained-release pellets comprise no further layers. In particular, no further active agent layer is applied to the sustained-release layer.

The sustained-release pellets are present in the multiple unit pellet tablet formulation in an amount of 30-70% by weight based on the total weight of the formulation (w/w), preferably 40-60% w/w.

As used herein "sustained release" refers to a technology used in pharmaceutical formulations such as tablets or capsules to release an active agent at a predetermined rate by maintaining a constant drug level for a specific period of time, preferably with minimum side effects. Preferably, sustained release is the release of active ingredient at such a rate that the blood levels are maintained within the therapeutic range but below toxicity levels over an extended period of time, e.g. 10 hours to 24 hours or longer. According to the present invention the blood levels are maintained within the therapeutic range but below toxicity levels for at least 6 h, preferably at least 8 h, more preferably at least 12 h.

Sustained release can be achieved through a variety of formulations, including matrix formulations, micro-encapsulation and OROS. The advantages of sustained-release tablets or capsules are that they can often be taken less frequently than immediate-release formulations of the same active agent, and that they keep steadier levels of the drug in the bloodstream.

The term "sustained release" as used herein is synonymous to the term "extended release" and is meant to include also "delayed release" which refers to pharmaceutical formulations that do not immediately disintegrate and release the active agents into the body. An example is an enteric coated pharmaceutical formulation, which dissolves in the intestine rather than the stomach.

The multiple unit pellet tablet formulation of the present invention comprises at least one opioid. Opioids are psychoactive chemical substances which act by binding to opioid receptors which are located principally in the central and peripheral nervous system and the gastrointestinal tract. Due to their analgesic effect opioids are used to treat acute pain such as post-operative pain. In addition, they are used in palliative care to alleviate the severe, chronic, disabling pain of terminal conditions such as cancer, and degenerative conditions such as rheumatoid arthritis.

It should be noted that the terms "opioid", "opioid analgesic" and "opioid agonist" are used interchangeably herein.

Examples of opioids that can be used in the multiple unit pellet tablet formulation of the present invention include without limitation µ-opioid receptor agonists such as alfentanil, buprenorphine, codeine, fentanyl, hydrocodone, hydromorphone, levomethadone, methadone, morphine, nalbuphine oxycodone, oxymorphone, pethidine, piritramid, remifentanil, sufentanil, tapentadol, tilidin, tramadol, and pharmaceutically acceptable salts thereof.

Preferably, an opioid exhibiting a dual mechanism of action as an agonist at the µ-opioid receptor and as a monoamine reuptake inhibitor is used according to the present invention such as tapentadol and tramadol or a pharmaceutically acceptable salt thereof.

More preferably, the opioid is tapentadol or a pharmaceutically acceptable salt thereof, e.g. tapentadol hydrochloride. In one embodiment of the invention tapentadol or a salt thereof is present as the only active agent.

In a preferred embodiment of the present invention the multiple unit pellet tablet formulation for oral administration comprises a plurality of sustained-release pellets and one or more pharmaceutically acceptable excipients, wherein the sustained-release pellets comprise, as an active agent, tapentadol or a salt thereof and are characterized by the following structure:
a) a neutral core,
b) an active agent layer applied to the neutral core comprising as an active agent tapentadol or a salt thereof and
c) a sustained-release layer which is applied to the active agent layer.

According to the present invention it is further possible to include an opioid antagonist in the multiple unit pellet tablet formulation in addition to the opioid agonist. Examples of suitable opioid antagonists are naloxone and naltrexone.

If an opioid antagonist is present in the multiple unit pellet tablet formulation in addition to the opioid agonist, the opioid antagonist may be included in the active-agent containing pellets together with the opioid agonist. Alternatively, two types of active-agent containing pellets may be present, one type comprising only opioid agonist and the other type comprising only opioid antagonist. The resulting active agent-containing pellets are coated with a sustained-release layer as described above in order to obtain sustained-release pellets.

In order to be effective in the treatment of pain an analgesically effective amount of the at least one opioid or salt thereof is required. In the multiple unit pellet tablet formulation of the present invention the at least one opioid or salt thereof is present in an amount of 2% to 50% by weight based on the total weight of the formulation (w/w), preferably 10-30% w/w.

Pharmaceutically acceptable salts of the opioid agonist and/or antagonist are known to a person skilled in the art or could be determined based on routine experimentation. Salts of the opioid include, for example, salts obtained from combination with an organic base, a mineral acid or an organic acid. Examples of such organic bases include, for example, trimethylamine, triethylamine and the like. Suitable acid addition salts can be obtained from the treatment with a mineral acid that include, for example, hydrochloric acid, hydrobromic acid, phosphoric acid and sulfuric acid, or with an organic acid that include, for example, ascorbic acid, citric acid, tartaric acid, lactic acid, maleic acid, malonic acid, fumaric acid, glycolic acid, succinic acid, propionic acid, acetic acid and methane sulfonic acid. In one embodiment, the salt of the opioid agonist and/or antagonist is a hydrochloride.

Suitable pharmaceutically acceptable excipients that can be used to prepare the multiple unit pellet tablet formulation of the present invention are well known to a person skilled in the art.

The multiple unit pellet tablet formulation according to the present invention may further be coated as a whole after being compressed. Suitable tablet coatings may be polymer and polysaccharide based and may further include plasticizers and pigments. Examples of suitable film-forming agents to be used in tablet coatings include without limitation hydroxypropyl methylcellulose, hydroxypropyl cellulose, polyvinyl acetate, polyvinyl alcohol and polyvinyl pyrrolidon.

Coatings provide for a smoother finish of tablets and make large tablets easier to swallow. In addition, tablet coatings are useful to extend the shelf-life of components that are sensitive to moisture or oxidation.

According to the present invention it is not intended to use special coatings such as enteric coatings or controlled-release coatings which should not be broken before use for coating the multiple unit pellet tablet as a whole. If the tablet is split, this would expose the tablet core to the digestive juices, circumventing the intended delayed-release effect.

The multiple unit pellet tablet formulation according to the present invention further comprises at least one retardant. Preferably, the at least one retardant is a cellulose ether. More preferably, the multiple unit pellet tablet formulation comprises at least two different retardants and not more than three different retardants. Preferably the at least two and not more than three different retardants are at least two different cellulose ethers and not more than three different cellulose ethers. Suitable cellulose ethers are selected from the group consisting of hydroxypropyl cellulose, hydroxypropyl methylcellulose, methyl cellulose, and ethyl cellulose. If the multiple unit pellet tablet formulation comprises two different cellulose ethers ethyl cellulose and hydroxypropyl cellulose are preferred.

The multiple unit pellet tablet formulation according to the present invention further comprises at least one plasticizer which is suitable for use with a retardant, preferably which is suitable for use with cellulose ethers e.g. propylene glycol, triacetin, triethyl citrate, dibutyl phthalate, diethyl phthalate, dibutyl sebacate, diethyl sebacate. The at least one plasticizer is present in the multiple unit pellet tablet formulation in an amount of 0.2-10.0% by weight based on the total weight of the formulation (w/w), preferably 1.0-3.5% w/w.

The multiple unit pellet tablet formulation according to the present invention optionally further comprises at least one pharmaceutically acceptable functional excipient. Functional excipients are well known in the art.

Non-limiting examples of functional excipients are binders such as polyvinylpyrrolidon, hydroxypropyl methylcellulose, hydroxypropyl cellulose; disintegrants such as sodium carboxymethyl cellulose, crospovidone; free-flow regulators such as silica colloidal anhydrous, talc; and lubricants such as magnesium stearate, calcium stearate, sodium stearyl fumarate, stearic acid.

In one embodiment the multiple unit pellet tablet formulation according to the present invention further comprises at least one retardant, at least one plasticizer and optionally at least one pharmaceutically acceptable functional excipient.

The invention further relates to sustained-release pellets comprising as an active agent at least one opioid or a salt thereof and characterized by the following structure: a) a neutral core, b) an active agent layer applied to the neutral core comprising as an active agent at least one opioid or a salt thereof and c) a sustained-release layer which is applied to the active agent layer. According to the present invention the sustained-release pellets comprise no further layers. In particular, no further active agent layer is applied to the sustained-release layer.

In particular, the sustained-release pellets of the present invention comprise at least one opioid selected from the group consisting of alfentanil, buprenorphine, codeine, fentanyl, hydrocodone, hydromorphone, levomethadone, methadone, morphine, nalbuphine oxycodone, oxymorphone, pethidine, piritramid, remifentanil, sufentanil, tapentadol, tilidin, tramadol, and pharmaceutically acceptable salts thereof. Preferably, the opioid is tapentadol or a pharmaceutically acceptable salt thereof, e.g. tapentadol hydrochloride.

The sustained-release pellets are preferably coated with a sustained-release layer comprising at least one cellulose ether, preferably at least two different cellulose ethers and not more than three different cellulose ethers as a retardant. If the sustained-release layer comprises two different cellulose ethers, ethyl cellulose and hydroxypropyl cellulose are preferred.

In one embodiment of the invention the sustained-release layer of the sustained-release pellets does not comprise vinyl polymers.

In order to prepare the sustained-release pellets for use in the multiple unit pellet tablet formulation of the present invention at least one organic solvent is used. Preferably, not more than two different organic solvents are used. Preferred suitable organic solvents are selected from ethanol, isopropanol and acetone. It is particularly preferred to use 96% ethanol as the only organic solvent.

The active agent-containing pellets are suitable for the preparation of the multiple unit pellet tablet formulations of the present invention. Therefore, the sustained-release pellets and optionally one or more pharmaceutically acceptable excipients are compressed into a tablet. This may be achieved by direct compression.

The multiple unit pellet tablet formulation of the present invention is suitable for use for analgesia in mammals, preferably in humans. Therefore, the present invention provides a multiple unit pellet tablet formulation for use in the treatment pain, preferably for use in the treatment of acute or chronic moderate to severe pain.

In one embodiment the present invention relates to the use of the multiple unit pellet tablet formulation for the treatment of pain.

The following examples are presented to further illustrate the invention and should not be construed as limiting the invention in any way.

### Example 1

The opioid is dissolved or suspended in an aqueous cellulose ether solution and the resulting solution/suspension sprayed onto cellulosic starter pellets (neutral core) by means of a fluidized bed pellet-coater. The resulting active agent pellets are then coated with a sustained-release layer using an alcoholic cellulose ether suspension or solution comprising a plasticizer by means of a fluidized bed pellet-coater. The resulting sustained-release pellets are dry-mixed with the remaining solid excipients in a bin blender with the lubricant being added and mixed separately at last. The resulting final blend is being compressed into tablets. Optionally, the remaining multiple unit pellet tablets are further coated as a whole using a non-functional coating e.g. hydroxypropyl methylcellulose, hydroxypropyl cellulose, polyvinyl acetate, polyvinyl alcohol or polyvinylpyrrolidone.

Suitable amounts of components used for the multiple unit pellet tablets are displayed in Table 1.

**Table 1**

| Components of the active agent-containing pellets | % by weight of multiple unit pellet tablet |
|---|---|
| Tapentadol*HCl | 11.6 |
| Hypromellose 5cP | 2.0 |
| Cellets-Pellets 212-350µm | 17.1 |
| Water* | q.s. |

| Components of the sustained-release pellets | % by weight of multiple unit pellet tablet |
|---|---|
| Active agent-containing pellets | 30.7 |
| Ethylcellulose 45cP | 11.8 |
| Hydroxypropylcellulose 300-600cP | 3.4 |
| Propylene glycol | 1.7 |
| Ethanol 96%* | q.s. |

| Components of the tablet core | % by weight of multiple unit pellet tablet |
|---|---|
| Sustained-release pellets | 47.6 |
| Microcrystalline Cellulose 180µm | 25.5 |
| Microcrystalline Cellulose 100µm | 25.5 |
| Crospovidone 170kPa | 1.3 |
| Silica colloidal anhydrous 200m²/g | 0.13 |
| Magnesium stearate | 0.13 |
| Total weight of multiple unit pellet tablet | 432 mg |

### Example 2

The opioid is dissolved or suspended in an aqueous cellulose ether solution and the resulting solution/suspension sprayed onto cellulosic starter pellets (neutral core) by means of a fluidized bed pellet-coater. The resulting active agent pellets are then coated with a sustained-release layer using an alcoholic cellulose ether suspension or solution comprising a plasticizer by means of a fluidized bed pellet-coater. The resulting sustained-release pellets are dry-mixed with the remaining solid excipients in a bin blender with the lubricant being added and mixed separately at last. The resulting final blend is being compressed into tablets. Optionally, the remaining multiple unit pellet tablets are further coated as a whole using a non-functional coating e.g. hydroxypropyl methylcellulose, hydroxypropyl cellulose, polyvinyl acetate, polyvinyl alcohol or polyvinylpyrrolidone.

Suitable amounts of components used for the multiple unit pellet tablets are displayed in Table 2.

**Table 2**

| Components of the active agent-containing pellets | % by weight of multiple unit pellet tablet |
|---|---|
| Tapentadol*HCl | 30.0 |
| Hypromellose 5cP | 3.0 |
| Cellets-Pellets 212-350µm | 0.3 |
| Water* | q.s. |

| Components of the sustained-release pellets | % by weight of multiple unit pellet tablet |
|---|---|
| Active agent-containing pellets | 33.3 |
| Ethylcellulose 45cP | 11.7 |
| Hydroxypropylcellulose 300-600cP | 3.3 |
| Propylene glycol | 1.7 |
| Ethanol 96%* | q.s. |

| Components of the tablet core | % by weight of multiple unit pellet tablet |
|---|---|
| Sustained-release pellets | 50.0 |
| Microcrystalline Cellulose 180µm | 24.3 |
| Microcrystalline Cellulose 100µm | 24.3 |
| Crospovidone 170kPa | 1.3 |
| Silica colloidal anhydrous 200m²/g | 0.13 |
| Magnesium stearate | 0.13 |
| Total weight of multiple unit pellet tablet | 167 mg |

| | |
|---|---|
| * evaporated after drying | |

## Claims

1. Multiple unit pellet tablet formulation for oral administration comprising a plurality of sustained-release pellets and one or more pharmaceutically acceptable excipients, wherein the sustained-release pellets comprise as an active agent at least one opioid or a salt thereof.

2. The formulation according to claim 1, wherein the sustained-release pellets are **characterized by** the following structure: a) a neutral core, b) an active agent layer applied to the neutral core comprising as an active agent at least one opioid or a salt thereof and c) a sustained-release layer which is applied to the active agent layer.

3. The formulation according to claim 1 or claim 2, wherein the at least one opioid is selected from the group consisting of alfentanil, buprenorphine, codeine, fentanyl, hydrocodone, hydromorphone, levomethadone, methadone, morphine, nalbuphine oxycodone, oxymorphone, pethidine, piritramid, remifentanil, sufentanil, tapentadol, tilidin, tramadol, and pharmaceutically acceptable salts thereof, preferably wherein the at least one opioid is tapentadol or a pharmaceutically acceptable salt thereof.

4. The formulation according to any one of claims 1 to 3 further comprising at least one opioid antagonist.

5. The formulation according to any one of claims 1 to 4, wherein the at least one opioid is present in an amount of 2% to 50% by weight based on the total weight of the formulation.

6. The formulation according to any one of claims 1 to 5, comprising at least one retardant, at least one plasticizer and optionally at least one pharmaceutically acceptable functional excipient.

7. The formulation according to claim 6, wherein the at least one retardant is a cellulose ether selected from the group consisting of hydroxypropyl cellulose, hydroxypropyl methylcellulose, methyl cellulose, and ethyl cellulose.

8. The formulation according to claim 6 comprising at least two retardants, wherein the retardants are cellulose ethers selected from the group consisting of hydroxypropyl cellulose, hydroxypropyl methylcellulose, methyl cellulose, and ethyl cellulose.

9. Sustained-release pellets comprising as an active agent at least one opioid or a salt thereof and **characterized by** the following structure: a) a neutral core, b) an active agent layer applied to the neutral core comprising as an active agent at least one opioid or a salt thereof and c) a sustained-release layer which is applied to the active agent layer.

10. The sustained-release pellets according to claim 9, wherein the at least one opioid is selected from the group consisting of alfentanil, buprenorphine, codeine, fentanyl, hydrocodone, hydromorphone, levomethadone, methadone, morphine, nalbuphine oxycodone, oxymorphone, pethidine, piritramid, remifentanil, sufentanil, tapentadol, tilidin, tramadol, and pharmaceutically acceptable salts thereof, preferably wherein the at least one opioid is tapentadol or a pharmaceutically acceptable salt thereof.

11. The sustained-release pellets according to claim 9 or claim 10, wherein the sustained-release layer which is applied to the active agent layer comprises ethyl cellulose and hydroxypropyl cellulose as retardants.

12. The sustained-release pellets according to any one of claims 9-11, wherein the sustained-release pellets and optionally one or more pharmaceutically acceptable excipients are compressed into a tablet.

13. The multiple unit pellet tablet formulation according to any one of claims 1-8 or the sustained-release pellets according to any one of claims 9-12 for use in the treatment of pain.

14. Use of the multiple unit pellet tablet formulation according to any one of claims 1-8 or the sustained-release pellets according to any one of claims 9-12 for the treatment of pain.
